# EUROPÄISCHE PATENTANMELDUNG

(11) **EP 2 656 770 A1**
(43) Veröffentlichungstag der Anmeldung: **30.10.2013**
(21) Anmeldenummer: 12401075.2
(22) Anmeldetag: 27.04.2012
(51) Int. Cl.: A47L 15/22, B08B 3/04, A61B 19/00, A47L 15/50

(54) **Reinigungs- und Desinfektionsautomat**

(71) Anmelder: Miele & Cie. KG, 33332 Gütersloh (DE)
(72) Erfinder: Heitmann, Michael, 33739 Bielefeld (DE); Schröder, Frank, 32139 Spenge (DE)

(57) **Zusammenfassung**

Die Erfindung betrifft einen Reinigungs- und Desinfektionsautomat insbesondere für medizinische Instrumente und Geräte, mit einem einen Spülraum (4) bereitstellenden Spülbehälter (3) und einer im Spülraum (4) angeordneten Sprüheinrichtung zur Beschickung von Spülgut mit Spülflotte. Um einen Reinigungs- und Desinfektionsautomat vorzuschlagen, der bei gleichbleibenden Außenabmessungen eine Kapazitätserweiterung bietet, wird mit der Erfindung ein Reinigungs- und Desinfektionsautomat der eingangs genannten Art vorgeschlagen, der sich dadurch auszeichnet, dass der Spülraum (4) in seiner Grundfläche quadratisch ausgebildet ist.

## Beschreibung

Die Erfindung betrifft einen Reinigungs- und Desinfektionsautomat insbesondere für medizinische Instrumente und Geräte, mit einem einen Spülraum bereitstellenden Spülbehälter und einer im Spülraum angeordneten Sprüheinrichtung zur Beschickung von Spülgut mit Spülflotte.

Reinigungs- und Desinfektionsautomaten der vorgeschriebenen Art sind aus dem Stand der Technik an sich gut bekannt, beispielsweise aus der DE 10 2009 009 768 A1 und der DE 10 2006 009 787 A1. Sie verfügen über einen Spülbehälter, der einen Spülraum bereitstellt. Im bestimmungsgemäßen Verwendungsfall dient der Spülraum der Aufnahme von zu reinigendem und/oder zu desinfizierendem Spülgut, bei dem es sich beispielsweise um medizinische Instrumente und Geräte handeln kann. Der Spülraum ist über eine Beschickungsöffnung des Spülbehälters zugänglich, die mittels einer verschwenkbar am Spülbehälter angeordneten Tür fluiddicht verschließbar ist.

Typischerweise kommt im bestimmungsgemäßen Verwendungsfall zur Spülgutreinigung ein Spülkorb zum Einsatz. Dieser wird mit dem zu reinigenden Spülgut bestückt und anschließend verwenderseitig durch die Beschickungsöffnung in den Spülraum eingeführt. Nach erfolgreich durchgeführter Spülung, dass heißt Reinigung und/oder Desinfektion des Spülgutes kann der Spülkorb mit dem davon aufgenommenen Spülgut verwenderseitig dem Spülraum durch die Beschickungsöffnung wieder entnommen werden.

Zur Spülgutpositionierung innerhalb des Spülkorbs können sogenannte Siebschalen zum Einsatz kommen. Dabei stellt ein Spülkorb typischerweise eine Vielzahl von in Höhenrichtung übereinander angeordneten Spülkorbebenen bereit, wobei jede Ebene mit einer bestimmten Anzahl von Siebschalen bestückt werden kann. Typische aus dem Stand der Technik bekannte Siebschalen haben eine Abmessung von 256 mm x 474 mm. Bei den gegebenen Außenabmessungen eines Reinigungs- und Desinfektionsautomatens können bei dieser Siebschalengröße drei Siebschalen je Spülkorbebene vorgesehen sein.

Innerhalb des Spülraums ist eine Sprüheinrichtung angeordnet. Diese dient der Beschickung von Spülgut mit Spülflotte. Typischerweise weist die Sprüheinrichtung Sprüharme auf, die verdrehbar gelagert ausgebildet sind. Es kann dabei zwischen automatenseitigen Sprüharmen einerseits und korbseitigen Sprüharmen andererseits unterschieden werden. Dabei betreffen "automatenseitige Sprüharme" solche Sprüharme, die im Spülraum des Automaten installiert sind. In der Regel sind zwei solcher Sprüharme vorgesehen, wobei der eine deckenseitig und der andere bodenseitig des Spülraums ausgebildet ist. "Korbseitige Sprüharme" betreffen indes solche Sprüharme, die am Spülkorb angeordnet sind, die also zusammen mit dem Spülkorb verwenderseitig verfahren werden können. Zum Anschluss dieser Sprüharme an ein Wasser- und/oder Spülflottenzuführsystem sind im Spülraum entsprechend ausgebildete Ankopplungsstellen vorgesehen, wie beispielsweise aus der DE 10 2007 003 894 A1 bekannt.

Zur Beschickung der Sprüheinrichtung mit Spülflotte kommt eine Umwälzpumpe zum Einsatz. Über diese wird im Spülraum befindliche Spülflotte angesaugt und über entsprechende Versorgungsleitungen und -rohre Sprüharmen und/oder sonstigen Fluidabgabeeinrichtungen der Sprüheinrichtung zugeführt. Diese Versorgungsleitungen und -rohre sind zur Maximierung der Größe des nutzbaren Spülraums außerhalb des Spülraums verlegt, wie sich dies beispielsweise aus der schon vorgenannten DE 10 2009 009 768 A1 beziehungsweise der vorgenannten DE 10 2006 009 787 A1 ergibt. Die Anordnung der Versorgungsleitungen außerhalb des Spülraums ist darüber hinaus mit Blick auf die schon vorbeschriebene Ankopplung von korbseitigen Sprüharmen an ein Fluidzuführungssystem von Vorteil.

Obgleich sich die vorgeschriebene Konstruktion im alltäglichen Praxiseinsatz bewährt hat, besteht Verbesserungsbedarf, insbesondere mit Blick auf eine verbesserte Kapazitätsausnutzung.

Es ist deshalb ausgehend von dem Vorgeschriebenen die **Aufgabe** der Erfindung, einen neuartigen Reinigungs- und Desinfektionsautomaten vorzuschlagen, der bei gleichbleibenden Außenabmessungen eine Kapazitätserweiterung bietet.

Zur **Lösung** dieser Aufgabe wird mit der Erfindung ein Reinigungs- und Desinfektionsautomat der eingangs genannten Art vorgeschlagen, der sich dadurch auszeichnet, dass der Spülraum in seiner Grundfläche quadratisch ausgebildet ist.

Aus dem Stand der Technik vorbekannte Reinigungs- und Desinfektionsautomaten verfügen über einen Spülraum mit rechteckförmiger Grundfläche, wobei zumeist die Breitenrichtung die kürzere Seite darstellt.

Die rechteckförmige Grundfläche ergibt sich konstruktionsbedingt. In seinen Außenabmessungen ist der Reinigungs- und Desinfektionsautomat in aller Regel in etwa quadratisch ausgebildet. Dieser insgesamt zur Verfügung stehende Bauraum dient einerseits zur Aufnahme des den Spülraum bereitstellenden Spülbehälters sowie andererseits der Ausbildung eines Installationsraums. Dabei dient der Installationsraum dazu, die zur Wasser- beziehungsweise zur Spülflottenführung außerhalb des Spülraums vorgesehenen Versorgungsleitungen und -rohre aufzunehmen. Die Ausgestaltung dieses Installationsraums bedingt, dass der den Spülraum ausbildende Spülbehälter in seiner Grundfläche von den im Wesentlichen quadratischen Außenabmessungen des Reinigungs- und Desinfektionsautomaten abweichend rechteckförmig ausgebildet ist.

Mit der Erfindung wird nun vorgeschlagen, vom bisherigen Konstruktionsprinzip abweichend auch für die Grundfläche des Spülraums eine im Wesentlichen quadratische Ausgestaltung vorzusehen. Dies wird konstruktiv dadurch erreicht, dass das zur Versorgung der Sprüheinrichung mit Spülflotte vorgesehene Versorgungsrohr innerhalb des Spülraums verlegt wird. Der bislang für die außerhalb des Spülraums verlegten Versorgungsleitungen und -rohre vorgesehene Installationsraum kann deshalb entfallen, was es gestattet, den Spülbehälter und damit auch den vom Spülbehälter bereitgestellten Spülraum zu vergrößern. In vorteilhafter Weise schafft dies eine Kapazitätserweiterung. Insbesondere können anstatt drei Siebschalen der standardisierten Größe nunmehr vier solcher Siebschalen je Spülkorbebene aufgenommen werden. Dies entspricht einer Kapazitätserweiterung von 33 Prozent. Die Außenabmessungen des Reinigungs- und Desinfektionsautomaten bleiben dabei unverändert im Unterschied zum Stand der Technik.

In der Fachwelt herrschte bislang das Vorurteil vor, dass zur Maximierung des nutzbaren Spülraums Versorgungsleitungen und -rohre außerhalb des Spülraumes zu verlegen sind. Die nunmehr mit der Erfindung vorgeschlagene konstruktive Ausgestaltung beschreitet einen völlig anderen Lösungsweg. Unter Wegfall des bislang erforderlichen Installationsraums wird der vom Spülbehälter bereitgestellte Spülraum in seiner Grundfläche quadratisch ausgebildet. Zur Versorgung der Sprüheinrichtung mit Spülflotte wird ein zentrales Versorgungsrohr vorgesehen, dass innerhalb des Spülraums angeordnet ist und sich bevorzugter Weise entlang der Mittelachse des Spülbehälters erstreckt. Je Korbebene sind die davon jeweils aufgenommenen Siebschalen kreisringförmig um jeweils 90 Grad versetzt hintereinander anzuordnen, womit zur Hindurchführung des zentralen Versorgungsrohres eine Anordnung der Siebschalen unter Freihaltung eines mittleren Bereichs erfolgt. In der Konsequenz dieser Ausgestaltung können im Unterschied zum Stand der Technik nicht nur drei Siebschalen der standardisierten Größe 256 mm x 474 mm je Ebene vorgesehenen werden, sondern vier solcher Siebschalen. Dies erbringt eine Steigerung der Aufnahmekapazität um 33 Prozent.

Die Sprüheinrichtung weist bevorzugter Weise eine Mehrzahl von um eine gemeinsame Drehachse verdrehbar ausgebildeten Sprüharmen auf. Dabei fällt bevorzugter Weise die Mittelachse des Spülbehälters mit der Drehachse der Sprüharme zusammen, womit sich das zentrale Verteilrohr entlang der Drehachse der Sprüharme innerhalb des Spülraums erstreckt.

Die erfindungsgemäße Ausgestaltung erbringt noch weitere Vorteile. Die Sprüharmlänge ist bei aus dem Stand der Technik bekannten Automaten entsprechend der kürzeren Seite des rechteckförmig ausgestalteten Spülraums auszubilden, was in nachteiliger Weise zur Folge hat, dass die Eckenbereiche und die Endbereiche der Längsseiten nur unzureichend mit Spülflotte versorgt werden können. Diese Problematik wird mit der erfindungsgemäßen Konstruktion überwunden, da eine insgesamt verbesserte Erreichbarkeit aller Spülraumbereiche für die von der Sprüheinrichtung abgegebenen Sprühstrahlen sichergestellt ist.

Die bislang vorgesehene äußere Spülflotten- beziehungsweise Wasserführung, dass heißt die Führung der Spülflotte über außerhalb des Spülraums ausgebildete Versorgungsleitungen und -rohre macht einerseits Umlenkungen sowie andererseits verzweigte Leitungs- und Rohrwege erforderlich. Damit einhergehend sind Strömungsverluste und Leckageprobleme. Mit der erfindungsgemäßen Ausgestaltung wird hier Abhilfe geschaffen. Mit dem zentral im Spülraum angeordneten Versorgungsrohr kann auf Umlenkungen und verzweigte Wasserwege dem Grunde nach vollständig nicht verzichtet werden, womit einerseits Strömungsverluste vermieden und andererseits Leckageprobleme auf ein Minimum reduziert werden können.

Es kann darüber hinaus auf Bypass-Spülungen für entlegene Ablaufelemente und - schläuche verzichtet werden, da derartige entlegene Ablaufelemente und -schläuche bei der erfindungsgemäßen Konstruktion nicht mehr vorhanden sind.

Weitere Vorteile und Merkmale der Erfindung ergeben sich aus den nachfolgenden Beschreibungen anhand der Figuren. Dabei zeigen:
- Figur 1: in einer schematischen Ansicht einen Reinigungs- und Desinfektionsautomaten nach der Erfindung;
- Figur 2: in einer Draufsicht von oben eine nach der erfindungsgemäßen Konstruktion mögliche Siebschalenanordnung und
- Figur 3: in einer Draufsicht von oben eine nach dem Stand der Technik mögliche Siebschalenanordnung.

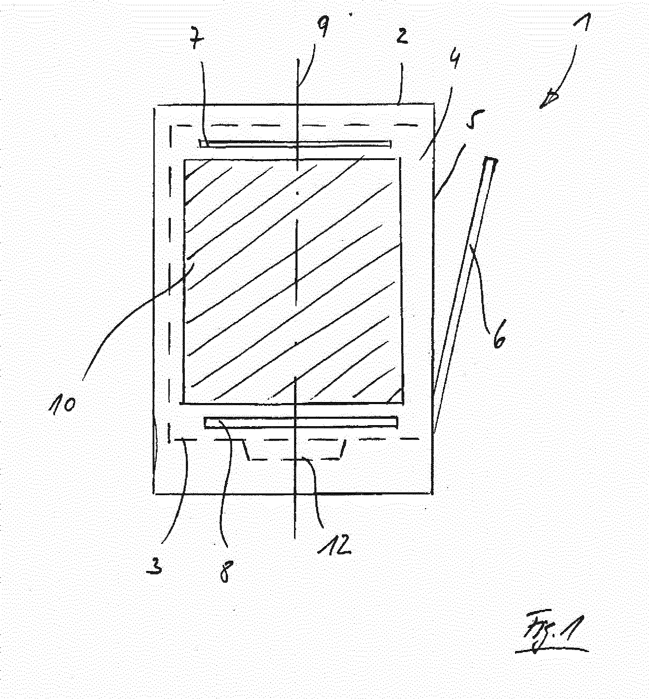

Figur 1 lässt in schematischer Ansicht einen Reinigungs- und Desinfektionsautomaten nach der Erfindung erkennen, im Nachfolgenden Spülautomat 1 genannt.

Der Spülautomat 1 nach der Erfindung ist als Gewerbeautomat ausgebildet und dient insbesondere der Behandlung von medizinischen Instrumenten und Geräten, beispielsweise Glaskolben, Reagenzgläsern und Schläuchen und/oder dergleichen.

Der Spülautomat 1 verfügt über ein Gehäuse 2. Innerhalb des Gehäuses 2 ist ein Spülbehälter 3 angeordnet, der einen Spülraum 4 bereitstellt. Der Spülraum 4 ist über eine Beschickungsöffnung 5 zugänglich, welche Beschickungsöffnung mittels einer Spülraumtür 6 fluiddicht verschließbar ist.

Im bestimmungsgemäßen Verwendungsfall dient der Spülraum 4 der Aufnahme von zu reinigendem Spülgut, beispielsweise medizinischen Instrumenten und Geräten. Zur Positionierung des zu reinigenden Spülguts innerhalb des Spülraums 4 dient ein Spülkorb 10. Dieser kann verwenderseitig aus dem Spülraum 4 herausverfahren beziehungsweise in diesen eingebracht werden.

Zum Zwecke der Beschickung des Spülraums 4 mit Spülflotte oder Trocknungsluft kommt eine Sprüheinrichtung zum Einsatz. Diese verfügt einerseits über automatenseitige Sprüharme sowie anderseits über korbseitige Sprüharme. Gemäß der schematischen Darstellung nach Figur 1 sind lediglich die automatenseitigen Sprüharme 7 und 8 dargestellt. Dabei ist der automatenseitige Sprüharm 7 deckenseitig des Spülraumes 4 montiert, wohingegen der weitere Sprüharm 8 als bodenseitiger Sprüharm 8 ausgebildet ist. Beide Sprüharme 7 und 8 drehen um die gemeinsame Drehachse 9.

Der Spülraum 4 mündet bodenseitig in einen Sammeltopf 12 ein. Zum Zwecke der Umwälzung der im bestimmungsgemäßen Verwendungsfall im Spülraum 4 befindlichen Spülflotte dient eine Umwälzpumpe 15. Diese saugt die sich im Sammeltopf 12 ansammelnde Spülflotte an und fördert diese zur Sprüheinrichtung, von wo aus dann eine Beschickung von Spülgut mit Spülflotte folgt.

Der Spülkorb 10 stellt typischerweise eine Mehrzahl von in Höhenrichtung übereinander angeordneten Korbebenen 13 bereit. Diese dienen jeweils der Aufnahme von zu spülendem Spülgut, wobei zur Aufnahme des Spülguts Siebschalen 14 vorgesehen sein können, die im bestimmungsgemäßen Verwendungsfall in den Spülkorb eingebracht und auf die einzelnen Spülkorbebenen 13 verteilt werden.

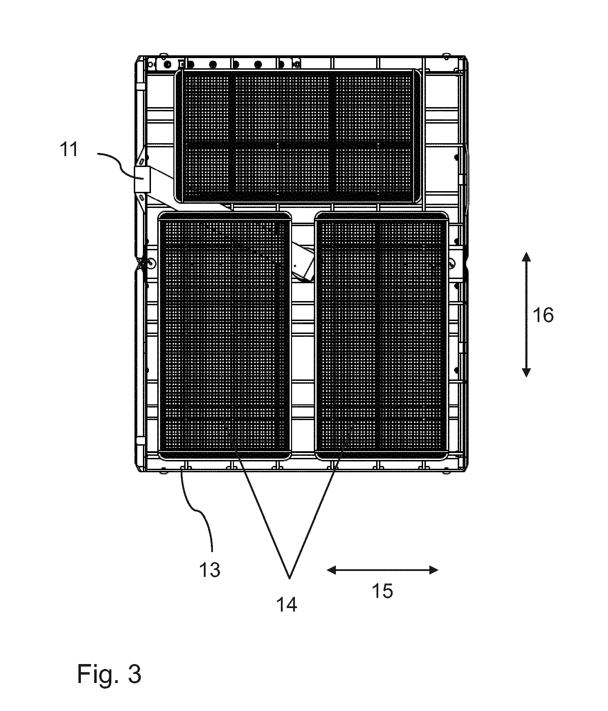

Figur 3 lässt in Draufsicht von oben eine Ausführungsform nach dem Stand der Technik erkennen, wobei dieser Darstellung eine Spülkorbebene 13 zu entnehmen ist, die mit insgesamt drei Siebschalen 14 der standardisierten Größe 256 mm x 474 mm bestückt ist.

Die Darstellung nach Figur 3 lässt ferner erkennen, dass ein spülkorbseitiger Sprüharm vorgesehen ist. Dieser ist über eine Anschlussstelle 11 an ein Spülflottenzuführsystem angeschlossen, welches über außerhalb des Spülraums 4 verlegte Versorgungsleitungen und -rohre verfügt. Die aus dem Stand der Technik bekannte Ausgestaltung betrifft eine in ihrer Grundfläche rechteckförmig ausgebildete Korbebene 13. Diese vermag im bestimmungsgemäßen Verwendungsfall drei der standardisierten Siebschalen 14 aufzunehmen. Die Korbebene 13 weist beispielsweise seine Größe von 790 mm in Tiefenrichtung 16 und von 636 mm in Breitenrichtung 15 auf.

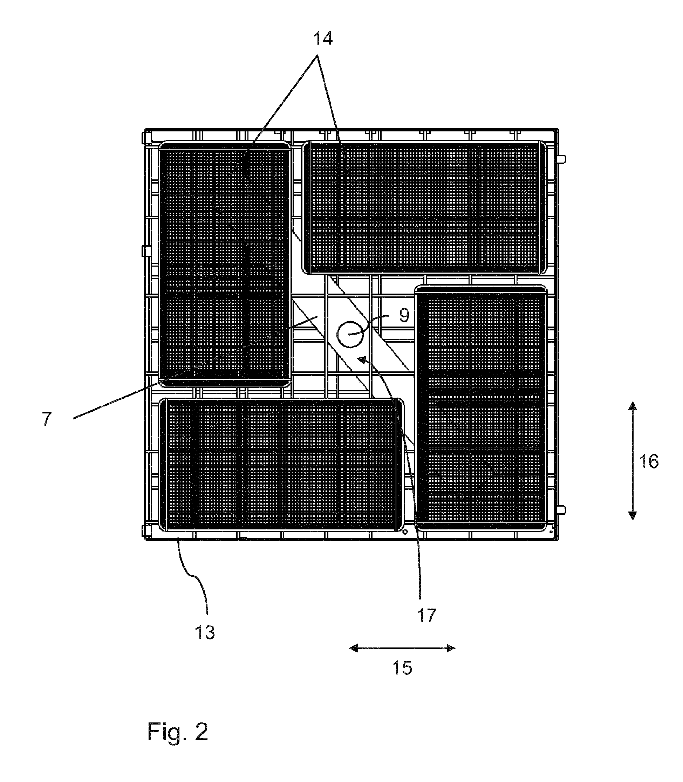

Nach der erfindungsgemäßen Ausgestaltung, die in Figur 2 gezeigt ist, ergibt sich ein Spülraum 4 und damit auch eine Korbebene 13, der beziehungsweise die in der Grundfläche im Wesentlichen quadratisch ausgebildet ist.

Gemäß dieser quadratischen Ausgestaltung können vier der standardisierten Siebschalen 14 je Korbebene 13 untergebracht werden, wobei diese um jeweils 90 Grad versetzt ringförmig hintereinander angeordnet sind. In der Konsequenz ergibt sich ein freigehaltener mittlerer Bereich 17 je Korbebene 13, um die herum die Siebschalen 14 positioniert sind. Dieser freigehaltene mittlere Bereich 17 dient der Hindurchführung eines zentralen und in den Figuren nicht näher dargestellten Versorgungsrohrs. Zu Versorgung der Sprüheinrichtung mit Spülflotte. Dabei erstreckt sich das Versorgungsrohr bevorzugter Weise entlang der Drehachse 9 der Sprüharme 7, 8 der Sprüheinrichtung, wie sich dies aus Figur 2 entnehmen lässt.

Wie sich aus Figur 2 ebenfalls ergibt, erlaubt es die quadratische Grundflächenausgestaltung, dass die Sprüharme 7 beziehungsweise 8 auch die Eckenbereiche gut erreichen können, was bei einer Ausgestaltung nach dem Stand der Technik nicht möglich ist, da aufgrund der nach dem Stand der Technik vorgesehenen rechteckförmigen Ausgestaltung die Sprüharme 7 und 8 in ihren geometrischen Abmessungen auf die jeweils kürzeste Seite der rechteckförmigen Grundfläche auszurichten sind. Die Kantenlänge der in Figur 2 gezeigten Korbebene 13 beträgt zwischen 750 mm und 800 mm, vorzugsweise zwischen 770 mm und 800 mm.

Bevorzugt ist indes eine Kantenlänge zwischen 790 mm und 800 mm, wobei "quadratisch" im Sinne der Erfindung auch eine Ausgestaltung meint, wonach die Kantenlängen in Breitenrichtung 15 einerseits und in Tiefenrichtung 16 um einige Millimeter voneinander abweichen, was konstruktions- und/oder fertigungsbedingt sein kann. Entscheidend für die Erfindung ist, dass in Abkehr von dem aus der aus dem Stand der Technik bekannten rechteckförmigen Ausgestaltung der Grundfläche eine im Wesentlichen quadratische Grundflächenausgestaltung erreicht ist, was aber keine im mathematischen Sinne exakte quadratische Ausgestaltung meint. So verfügt die in Figur 2 dargestellte Korbebene 13 über eine Abmessung von 790 mm in Tiefenrichtung 16 und 798 mm in Breitenrichtung.

### Bezugszeichenliste

- 1: Spülautomat
- 2: Gehäuse
- 3: Spülbehälter
- 4: Spülraum
- 5: Beschickungsöffnung
- 6: Spülraumtür
- 7: Sprüharm
- 8: Sprüharm
- 9: Drehachse
- 10: Spülkorb
- 11: Anschlussstelle
- 12: Sammeltopf
- 13: Korbebene
- 14: Siebschale
- 15: Breitenrichtung
- 16: Tiefenrichtung
- 17: Mittlerer Bereich

## Patentansprüche

1. Reinigungs- und Desinfektionsautomat insbesondere für medizinische Instrumente und Geräte, mit einem einen Spülraum (4) bereitstellenden Spülbehälter (3) und einer im Spülraum (4) angeordneten Sprüheinrichtung zur Beschickung von Spülgut mit Spülflotte, **dadurch gekennzeichnet, dass** der Spülraum (4) in seiner Grundfläche quadratisch ausgebildet ist.

2. Automaten nach Anspruch 1, **dadurch gekennzeichnet, dass** die Sprüheinrichtung eine Mehrzahl von um eine gemeinsame Drehachse (9) verdrehbar ausgebildeten Sprüharmen (7, 8) aufweist.

3. Automaten nach Anspruch 1 oder 2, **gekennzeichnet durch** ein sich entlang der Drehachse (9) der Sprüharme (7, 8) erstreckendes Versorgungsrohr zur Versorgung der Sprüharme (7, 8) mit Spülflotte.

4. Automat nach einem der vorhergehenden Ansprüche, **gekennzeichnet durch** einen im Spülraum (4) positionierbaren Spülkorb (10), der eine Korbebene (13) bereitstellt, die zur Grundfläche des Spülraumes (4) korrespondierend quadratisch ausgebildet ist.

5. Automat nach Anspruch 4, **dadurch gekennzeichnet, dass** die Korbebene (13) der Aufnahme von vier rechteckigförmigen Siebschalen (14) dient, die zur Hindurchführung des Versorgungsrohrs unter Freihaltung eines mittleren Bereichs (17) der Korbebene (13) positionierbar sind.

6. Automat nach Anspruch 5, **dadurch gekennzeichnet, dass** eine Siebschale (14) eine Abmessung von 256 mm x 474 mm aufweist.

7. Automat nach einem der vorhergehenden Ansprüche, **dadurch gekennzeichnet, dass** eine Korbebene (13) eine Kantenlänge zwischen 750 mm und 800 mm, vorzugsweise zwischen 770 mm und 800 mm, noch mehr bevorzugt zwischen 790 mm und 800 mm aufweist.
